Europäisches Patentamt

**European Patent Office**

Office européen des brevets

(19)

(11) Veröffentlichungsnummer: **0 008 432**
**B1**

(12) # EUROPÄISCHE PATENTSCHRIFT

(45) Veröffentlichungstag der Patentschrift:
29.09.82

(51) Int. Cl.³: **G 01 N 33/58**

(21) Anmeldenummer: 79102950.7

(22) Anmeldetag: 14.08.79

(54) Immunologisches Bestimmungsverfahren.

(30) Priorität: 17.08.78 DE 2836046

(43) Veröffentlichungstag der Anmeldung:
05.03.80 Patentblatt 80/5

(45) Bekanntmachung des Hinweises auf die Patenterteilung:
29.09.82 Patentblatt 82/39

(84) Benannte Vertragsstaaten:
AT BE CH DE FR GB IT LU NL SE

(56) Entgegenhaltungen:
DE-A-2 710 264
DE-A-2 746 250
FR-A-2 272 102
FR-A-2 272 398

Chemical Abstracts, Band 84, Nr. 17, 26. April 1976,
Seiten 383 und 384, Zusammenfassung 119756a.
Columbus, Ohio, USA
Svehag, S.E. «A solid-phase radioimmunoassay for
C1q-
binding immune complexes. I. △ IgG as indicator» &
Scand.
J. Immunol. 1975, Nr. 4, Seiten 687.97.

(73) Patentinhaber: BEHRINGWERKE
AKTIENGESELLSCHAFT, Postfach 1140,
D-3550 Marburg/Lahn (DE)

(72) Erfinder: Pernice, Walter, Dr., Zasiusstrasse 112,
D-7800 Freiburg/Breisgau (DE)
Erfinder: Sedlacek, Hans-Harald, Dr., Sonnenhang 3,
D-3550 Marburg/Lahn (DE)

(74) Vertreter: Meyer-Dulheuer, Karl-Hermann, Dr. et al,
HOECHST Aktiengesellschaft Zentrale Patentabteilung
Postfach 80 03 20, D-6230 Frankfurt/Main 80 (DE)

Chemical abstracts, Band 85, Nr. 13, 27. September
1976,
Seite 448, Zusammenfassung 91960g. Columbus, Ohio,
USA
Hay. F.C. et al. «Routine assay for the detection of
immune
complexes of known immunoglobulin class using solid
phase C1q».

& Clin. Exp. Immunol., 1976, Nr. 24, Seiten 396–400.

## Immunologisches Bestimmungsverfahren

Die Erfindung betrifft ein immunologisches Verfahren zur Bestimmung eines aus Antigenen und Antikörpern gebildeten Immunkomplexes in Flüssigkeiten.

Immunkomplexe sind Antigen-Antikörper-Verbindungen, die nicht nur in vitro, sondern auch in vivo bei vielen Krankheiten gebildet werden und in Körperflüssigkeiten auftreten. Bei dem Antigen eines Immunkomplexes kann es sich um einen Vertreter aus einer Vielfalt von Substanzen handeln. Diese werden häufig von Microorganismen, Viren, Bakterien, Parasiten, Protozoen gebildet und treten im Blutkreislauf von höheren Lebewesen auf. Es können jedoch auch Produkte von tierischem Gewebe oder von malignen Zellen sein, die als Antigene Anlass zur Antikörperbildung geben. Die von einem Antigen induzierten Antikörper sind im Hinblick auf das Antigen spezifisch. Die Antikörperbildung des lebenden Organismus führt zur Bildung von Antigen-Antikörperkomplexen, die als Immunkomplex bezeichnet werden. Immunkomplexe sind in vivo in der Lage, Komplementfaktoren zu binden. Immunkomplexe werden normalerweise rasch durch das retikuloendotheliale System aus dem Organismus eliminiert. Unter bestimmten Umständen persistieren Immunkomplexe im Körper und verursachen chronische Immunkomplexerkrankungen. Persistierende Immunkomplexe sind normalerweise derart zusammengesetzt, dass nicht alle immunologischen Bindungsstellen des Antigens mit Antikörpern abgesättigt sind. Der Nachweis von Immunkomplexen in den Körperflüssigkeiten liefert wertvolle Hinweise zur Diagnose von Krankheiten, die als Immunkomplexerkrankungen zusammengefasst werden können.

Bei derartigen Erkrankungen ist jedoch nicht nur der Nachweis von Immunkomplexen, sondern auch die Differenzierung der darin enthaltenen Antigene von grösstem Interesse. Es besteht demnach ein Bedarf an einem im Hinblick auf das Antigen differenzierenden Immunkomplexnachweis. Bislang war hierfür ein mit zwei Schritten zu kennzeichnendes Verfahren möglich, nämlich zunächst der Nachweis des Immunkomplexes und danach die Identifizierung des Antigens.

Es wurde nun gefunden, dass der Nachweis von Immunkomplexen und die Identifizierung des Antigens in einem einzigen Verfahren erreicht werden können.

Dazu werden spezifische, gegen das Antigen eines Immunkomplexes gerichtete Reaktanten A (in der Regel Antikörper) an einen Träger gebunden. Dieses beschichtete Trägermaterial wird mit der zu prüfenden Flüssigkeit in Kontakt gebracht. Danach wird der so behandelte Träger mit einer Lösung eines gegebenenfalls markierten Reaktanten B (Antikörper, Komplement), der gegen den im Immunkomplex vorhandenen Antikörper gerichtet ist, in Kontakt gebracht und schliesslich der gebundene oder der in der Flüssigkeit gelöste Anteil des Reaktanten B, vorzugsweise durch Messung des Markierungsmittels, bestimmt.

Im Falle eines markierten Reaktanten gibt die Menge des jeweils bestimmten Markierungsmittels die Menge der jeweils gebundenen Immunkomplexe an. Bei dem Markierungsmittel kann es sich beispielsweise um ein radioaktives Atom, ein Enzym, ein Coenzym oder um eine fluoreszierende Gruppe handeln.

Beiden Verfahren ist gemeinsam, dass die Antigenspezifität durch die Verwendung von antigenspezifischen Antikörpern gewährleistet ist. Beispielsweise benötigt man für den Nachweis HBsAg-haltiger Immunkomplexe Anti-HBsAg-Antikörper, für den Nachweis von Tetanustoxoid-haltigen Immunkomplexen Anti-Tetanustoxoid-Antikörper.

Alternativ zur Verwendung markierter Reaktanten können die trägergebundenen Immunkomplexe selbst durch Indikatorreaktionen nachgewiesen werden. Dies kann z.B. durch Messung der Komplementbindung oder durch Agglutination des Trägers erfolgen.

Gegenstand der Erfindung ist demnach ein immunologisches Bestimmungsverfahren für einen Immunkomplex in einer Flüssigkeit, dadurch gekennzeichnet, dass

a) ein für eine antigene Determinante des Antigens in dem Immunkomplex spezifischer, an einen Träger gebundener Raktant A in einer für die Bindung des Immunkomplexes ausreichenden Menge mit der immunkomplexhaltigen Flüssigkeit inkubiert wird;

b) der so behandelte Träger nach Abtrennung der Flüssigkeit mit einer für die Bindung an den Immunkomplex ausreichenden Menge einer Lösung eines gegebenenfalls markierten Reaktanten B inkubiert wird, der spezifisch für eine antigene Determinante des Antikörpers in dem Immunkomplex ist;

c) und nach Trennung von Träger und Flüssigkeit durch Messung des an den Träger gebundenen oder der in der Flüssigkeit verbliebenen Menge des Reaktanten B der Immunkomplex bestimmt wird.

An den Träger ist somit ein mit dem Antigen reagierender Reaktant A gebunden, während der Reaktant B spezifisch für den Antikörper des Immunkomplexes ist.

Allgemeine Verfahrensbeschreibung

Ein für das im Immunkomplex vorliegende Antigen spezifischer Reaktant A wird an einen festen Träger gebunden. Reaktanten spezifisch für das Antigen sind Antikörper oder deren antigenbindende Fragmente. Da Immunkomplexe meist schon in vivo Komplementfaktoren binden, können auch Anti-Komplementantikörper, z.B. Fcspezifische Reaktanten, verwendet werden.

Als Träger sind geeignet Formkörper, beispielsweise amorphe Partikel, Kugeln, Plättchen, Folien oder Reagenzgefässe aus anorganischem (z.B.

Glas) oder organischem Material (z. B. Homo- oder Copolymere von Vinylverbindungen wie Olefinen, Vinylacetat, Vinylchlorid, Vinylidenchlorid, Tetrafluoräthylen, Styrol, Acrylsäure oder Methacrylsäure, ferner Polymere von Formaldehyd und cyclischen Acetalen sowie aus Polykondensaten, wie Polyestern, Polycarbonaten oder Polyamiden oder vernetzte Kohlenhydrate und vernetzte Proteine), weiterhin biologische partikuläre Träger wie Zellen (z. B. Erythrozyten).

Die Bindung kann adhäsiv oder kovalent erfolgen. Zur adhäsiven Beschichtung wird der Träger mit dem Reaktanten in Kontakt gebracht. Beispielsweise wird der Reaktant in einer wässrigen Lösung, vorzugsweise in einem Puffer (z. B. phosphatgepufferter isotonischer Kochsalzlösung (PBS), pH 7,2), in einer Konzentration von 1 μg bis 1 g/l, vorzugsweise 10 μg/ml, gelöst, 1 Min. bis 5 Tage, vorzugsweise 24 Stunden lang, mit dem Träger stehen gelassen. Überschüssiges Reagenz wird durch Waschen, vorteilhaft mit Puffer, der zweckmässig ein Detergens enthält (z. B. PBS, pH 7,2 – Polyoxy–äthylensorbitanmonolaurat (Tween 20) 0,1%ig) entfernt. (Tween ist ein eingetragenes Warenzeichen.)

Kovalente Bindungen der jeweiligen Reaktanten werden mit den unterschiedlichen Trägern nach literaturbekannten Verfahren durchgeführt (z. B. Orth et al., Angw. Chemie, 1972, 84, S. 319 ff; Silman et al., Annu. Rev. Biochem., 1966, 35, S. 873 ff; Becht et al., J. Immunology, 1968, 101, S. 18 ff; Weetall, Marcel Dekker, Inc., New York, P. 32, 1975). Die Bestimmung der Immunkomplexe wird nun wie folgt durchgeführt:

Der mit dem jeweiligen Reaktanten A beschichtete Träger wird 1 Min. bis 48 Stunden, vorzugsweise 10 Stunden, mit der Immunkomplexe enthaltenden Flüssigkeit, vorzugsweise einer Serumprobe, inkubiert und zweckmässig zur Entfernung überschüssiger Serumbestandteile mit Puffer gewaschen.

Anschliessend wird der so behandelte Träger mit einer Lösung eines vorzugsweise markierten Reaktanten B, der spezifisch für den immunkomplexgebundenen Antikörper im Immunkomplex ist, 1 Min. bis 48 Stunden, vorzugsweise 1 Stunde lang, inkubiert. Reaktanten B, die spezifisch für den Antikörper sind, können mikrobielles Protein A, Komplement, Antikörper oder Antikörperfragmente sein, die gegen antigene Determinanten nativer oder denaturierter Antikörper gerichtet sind.

Falls der Immunkomplex in vivo Komplementfaktoren gebunden hat, können auch Antikörper oder Antikörperfragmente, die gegen Komplementfaktoren gerichtet sind, als antikörperspezifische Reaktanten verwendet werden.

Reaktanten, die spezifisch für das Antigen sind, sind antigenspezifische Antikörper oder Antikörperfragmente. Spezifische Antikörperpräparationen werden entweder aus den Seren spezifisch immunisierter Tiere oder auch aus menschlichen Seren isoliert (z. B. Anti-Desoxyribonukleinsäure-Antikörper aus Patientenseren mit Lupus erythematodes, Anti-Hepatitis-B-Antikörper aus Seren von Patienten mit abgelaufener Hepatitis, Rheumafaktoren aus Patientenseren mit rheumatoider Arthritis) (Lit.: Humphrey and White: Immunologie, Thieme-Verlag Stuttgart, 1972; Gell, Coombs, Lachmann: Clinical Aspects of Immunology, Blackwell, Oxford, London, Edinburgh, Melburne, 1975). Die im Test einzusetzenden optimalen Verdünnungen der Reaktanten dürfen keine unspezifische, d. h. ohne Anwesenheit von trägergebundenem Immunkomplex nachweisbare Verbindung mit dem Träger eingehen. Diese optimalen Verdünnungen sind für jede Reaktantencharge speziell durch Vorversuche zu ermitteln. Gebräuchliche Verdünnungen sind z. B. 1 : 10 bis 1 : 400. Zur Kontrolle dienen Versuche, die, wie oben beschrieben, jedoch mit Seren ohne Immunkomplexe durchgeführt werden.

Als Markierungsmittel für die Reaktanten werden, wie bereits erwähnt, beispielsweise radioaktive oder fluoreszierende Substanzen oder Enzyme bevorzugt.

Die Messung des Markierungsmittels am Träger kann mit den dem Fachmann geläufigen Methoden erfolgen.

Die Erfindung wird an nachstehenden Beispielen näher erläutert:

Beispiel 1:

Verwendung trägergebundener Reaktanten A, spezifisch für das Antigen Tetanustoxoid.

Immunkomplexe mit Tetanustoxoid als Antigen werden durch Mischen von Tetanus-Antikörpern vom Menschen mit Tetanustoxoid hergestellt.

Herstellung eines adsorptiv mit Antikörpern beschichteten Trägers:

In 400 μl fassenden Polystyrol-Röhrchen werden 100 μl einer $1,1 \times 10^{-3}$%igen Lösung (Gewicht Protein/Volumen phosphatgepufferter Kochsalzlösung) eines Anti-Tetanus-Immunglobulins vom Kaninchen eingefüllt und bei 4 °C gelagert. Nach 24 Stunden wird die Lösung abgesaugt. Die Röhrchen werden dreimal mit je 400 μl Waschlösung (phosphatgepufferter Kochsalzlösung, pH 7,2, enthaltend 0,1% (g/v) Tween 20) gewaschen.

1. Inkubation:

Die mit dem Anti-Tetanustoxoid beschichteten Röhrchen werden mit 0,1 ml der wie oben hergestellten, Immunkomplex enthaltenden Lösung gefüllt, 1 : 4 mit phosphatgepufferter Kochsalzlösung, enthaltend 5% Rinderserumalbumin, verdünnt, 10 Stunden bei 21 °C stehen gelassen. Danach wird die Lösung abgesaugt und wie bei der Beschichtung des Trägers gewaschen.

2. Inkubation:

Anschliessend wird in die Röhrchen 0,1 ml eines mit Peroxydase nach dem Verfahren von Nakane und Kawaoe, J. Histochem. Cytochem., 1972, 22, 1084) markierten Kaninchen-Anti-Human-Gammaglobulins in einer Verdünnung von 1 : 50 mit phosphatgepufferter Kochsalzlösung, enthaltend 5% Rinderserumalbumin, eingefüllt, und es wird dann nach 1 Stunde (21 °C) wieder wie bei der Beschichtung gewaschen.

## 3. Messung der Markierung:

0,1 ml einer frisch zubereiteten 0,1%igen o-Phenylendiamin-HCl-Lösung in Citrat-Phosphat-Puffer (5 ml 0,1 M Citronensäure + 5 ml 0,2 M $Na_2HPO_4$), versetzt mit 0,1 ml einer 1% (v/v) $H_2O_2$-Lösung wurden in die Röhrchen einpipettiert. Nach 30 bis 60 Minuten Reaktionszeit im Dunkeln wird die Reaktion mit 0,1 ml einer 2 N $H_2SO_4$-Lösung gestoppt und die Extinktion bei 490 nm mit Hilfe eines Beckman-Photometers gemessen.

| Tetanustoxoid-haltige Immunkomplexe | Milliextinktion |
|---|---|
| im geringen Antigenüberschuss | 2133 |
| im extrem hohen Antigenüberschuss | 635 |
| Kontrollen | |
| reines Tetanustoxoid ohne Immunkomplexe | 310 |
| reines Tetanusimmunserum ohne Immunkomplexe | 831 |

Immunkomplexe sind in Abhängigkeit von ihrer Zusammensetzung (Antigen/Antikörperverhältnis) nachweisbar, besonders gut jedoch im leichten Antigenüberschuss.

## Beispiel 2:

Verwendung trägergebundener Reaktanten A spezifisch für das Antigen HBsAg.

Patientenseren mit HBsAg-haltigen Immunkomplexen und 60 Normalseren wurden getestet.

Herstellung des adsorptiv mit Antikörpern beschichteten Trägers:

In 400 µl fassende Polystyrol-Röhrchen werden 100 µl einer $1,1 \times 10^{-3}$%igen Lösung (Gewicht Protein/Volumen, phosphatgepufferter Kochsalzlösung) eines Anti-Hepatitis-Immunglobulins vom Kaninchen, eingefüllt. Nach 24 Stunden (4 °C) wird die Lösung abgesaugt, dreimal mit je 400 µl phosphatgepufferter Kochsalzlösung, pH 7,2, enthaltend 0,1% (g/v) Tween 20, gewaschen.

## 1. Inkubation:

Die mit dem Kaninchen-Anti-Hepatitishyperimmunglobulin beschichteten Röhrchen werden mit 0,1 ml der Immunkomplexe enthaltenden Lösung gefüllt, 1 : 4 mit phosphatgepufferter Kochsalzlösung, enthaltend 5% Rinderserumalbumin, verdünnt, 10 Stunden bei 21 °C stehen gelassen.

Danach wird die Lösung abgesaugt und wie bei der Beschichtung des Trägers gewaschen.

## 2. Inkubation:

Anschliessend wird in die Röhrchen 0,1 ml eines mit Peroxydase nach dem Verfahren von Nakane und Kawaoe (J. Histochem. Cytochem., 1974, 22, 1084) markierten Kaninchen-Anti-Human-Gammaglobulins in einer Verdünnung von 1 : 50 mit phosphatgepufferter Kochsalzlösung, enthaltend 5% Rinderserumalbumin, eingefüllt, und es wird dann nach 1 Stunde (21 °C) wieder wie bei der Beschichtung des Trägers gewaschen.

## 3. Messung der Markierung wie bei Beispiel 1.

Die Ergebnisse sind in der folgenden Tabelle dargestellt

| Serum-Nr. | Diagnose | Immunkomplex (Milliextinktion) HBsAg nachgewiesen nach Beispiel 2 |
|---|---|---|
| 1 | Hepatitis B | 301 |
| 2 | Hepatitis B | 1107 |
| 3 | Hepatitis B | 1129 |
| 4 | chron. aggr. Hepatitis B | 1321 |
| 5–65 | 60 Normalseren | <200 |

In Seren von Patienten mit Hepatitis konnten eindeutig antigen-spezifisch Immunkomplexe nachgewiesen werden.

## Beispiel 3:

Verwendung von Polymethylmethacrylatfolie mit aktiven Azidgruppen.

Beschichtung der Folie:

Folienstücke gleicher Grösse mit aktiven Azidgruppen aus Polymethylenmethacrylat, hergestellt nach dem Verfahren von M. Lynn, Immobilized Enzymes, Antigens, Antibodies and Peptides, ED Howard H. Weetall, Marcel Dekker, Inc., New York, 1975, p. 32) werden mit einer 0,1%igen Lösung von Pferd-Anti-Tetanustoxoid-Immunglobulin in PBS, pH 7,2, inkubiert und zur Entfernung überschüssiger Antikörper dreimal mit PBS, pH 7,2, gewaschen.

## 1. Inkubation:

Die mit Pferd-Antitoxoid beschichtete Folie wird mit der Immunkomplexe (Immunkomplexe mit Tetanustoxoid als Antigen wie bei Beispiel 1) enthaltenden Testprobe 1 : 4 mit PBS-Albumin 5% verdünnt, 10 Stunden inkubiert. Danach wird die Folie dreimal mit Waschlösung wie in Beispiel 1 gewaschen.

## 2. Inkubation:

Nachfolgend wird die Folie mit Peroxydasemarkiertem Kaninchen-anti-Human-Gammaglobulin, 1 : 50 in PBS-BSA (s. Beispiel 1) verdünnt, 1 Stunde lang (21 °C) inkubiert, dreimal mit Waschlösung (s.o.) gewaschen und die Markierung auf der Folie wie bei Beispiel 1 gemessen.

Ergebnisse:

| | Tetanustoxoid ohne Immunkomplexe | Tetanustoxoid-haltige Immun-komplexe im geringen Antigen-überschuss | Tetanusimmun-serum ohne Immunkomplexe |
|---|---|---|---|
| Extinktion bei 420 nm | 208 | 803 | 245 |

In der Immunkomplex-haltigen Probe, hergestellt wie in Beispiel 1, konnten gegenüber Serum und Tetanustoxoid ohne Immunkomplexe eindeutig antigenspezifisch Immunkomplexe nachgewiesen werden.

Beispiel 4:
Verwendung von Polymethylmethacrylatfolie mit aktiven Azidgruppen für die kovalente Bindung von F(ab)$_2$ Fragmenten:
Beschichtung der Folie:
Polymethylmethacrylat-Folienstücke gleicher Grösse (s. Beispiel 3) werden mit einer 0,1%igen Lösung einer Anti-Tetanustoxoid F(ab)$_2$ Präparation vom Pferd in PBS, pH 7,2 (hergestellt nach dem Verfahren von Visonoff, A. et al. Arch. Biochem. Biophys. 89, 230 (1960)) 24 Stunden bei 21 °C inkubiert und zur Entfernung überschüssiger Reaktanten 3 mal mit Waschlösung (s. Beispiel 1) gewaschen.

1. Inkubation:
Die beschichtete Folie wird mit der Immunkomplexe enthaltenden Testprobe, die 1 : 4 in PBS, pH 7,2 und BSA 5% verdünnt wurde, 10 Stunden inkubiert und anschliessend 3 mal mit Waschlösung gewaschen.

2. Inkubation:
Nachfolgend wird die Folie in peroxydase-markiertem (s. Beispiel 1) Anti-human IgG vom Kaninchen, 1 : 50 verdünnt mit PBS, pH 7,2 und BSA 5%, 2 Stunden inkubiert und anschliessend 3 mal in Waschlösung (s. Beispiel 1) gewaschen.

Die Markierung auf der Folie wird gemessen, indem man die so vorbereiteten Folienstücke 30–60 Min. lang in 0,5 ml Substratlösung (O-Phenylendiamin · HCl-Lösung, s. Beispiel 1) inkubiert und nachfolgend die Extinktion der Lösung bei 490 nm misst.

Ergebnisse:

| Tetanustoxoid ohne Immunkomplexe | Tetanushyper-immunserum mit Immunkomplexen | Tetanushyper-immunserum ohne Immun-komplexe |
|---|---|---|
| 95 | 498 | 100 |

Gegenüber den Kontrollen ohne Immunkomplexe zeigt die Probe mit Immunkomplexen deutlich erhöhte Extinktionswerte.

Beispiel 5:
Nach dem Verfahren von Becht (J. Immunol. 101, S. 18, 1968) mit Sulfosalicylsäure stabilisierte und tannierte Erythrozyten, 2%ig in PBS, werden 24 Stunden bei 4 °C unter Schütteln mit Anti-tetanustoxoid-Immunglobulin 0,01%ig vom Pferd, inkubiert, anschliessend 3 mal in PBS, enthaltend 0,1% Tween 20, gewaschen und auf eine 1%ige Zellsuspension eingestellt.

1. Inkubation:
50 µl der Erythrozytensuspension werden mit 50 µl Tetanustoxoid-human-Antitoxoid-IC-haltigen Testproben, 1 : 4 verdünnt in PBS, Ph 7,2, 2 Stunden unter Schütteln inkubiert und anschliessend dreimal mit Waschlösung gewaschen.

2. Inkubation:
Das Zellsediment wird mit 100 µl frisch gewonnenem Meerschweinchenserum 1 : 16 verdünnt, unter Schütteln 30 Min. bei Zimmertemperatur inkubiert und dann so lange (10 Min./21 °C) stehen gelassen, bis sich die Erythrozyten am Röhrchenboden abgesetzt haben.

3. Messung des Komplementverbrauchs:
Frische Schaf-Erythrozyten werden dreimal mit PBS gewaschen. 1 ml Zellsediment wird mit 0,5 ml einer 1%igen CrCl$_3$-Lösung in PBS suspendiert und mit 0,5 ml Humanimmunglobulin 10% gemischt, nach 4 Minuten dreimal mit PBS gewaschen und auf eine 1%ige Zellsuspension eingestellt. Gleiche Volumina der 1%igen Zellsuspension und eines Kaninchen-Antihuman-IgG-Serums, 1%ig in PBS, werden zusammengegeben und 45 Minuten bei Zimmertemperatur unter Schütteln inkubiert.

50 µl dieser so erhaltenen Zellsuspension werden mit 50 µl des nach der 2. Inkubation erhaltenen Überstandes 30 Min. bei 37 °C stehen gelassen. Die Extinktion des zellfreien Überstandes wird bei 546 nm mit einem Beckman-Photometer gemessen. Niedrige Extinktionswerte zeigen einen hohen Verbrauch von Komplement im 2. Inkubationsschritt und damit einen hohen Gehalt der Proben an Immunkomplexen an; hohe Werte bedeuten, dass die Testproben wenig Immunkomplexe enthalten.

Ergebnisse:

| | Tetanustoxoid-Antikörper-IC im extr. Antigen-überschuss | Tetanustoxoid-Antikörper-IC im leichten Antigen-überschuss | Tetanustoxoid-Antikörper-IC im Antikörper-überschuss | Antikörper ohne Immunkomplex |
|---|---|---|---|---|
| Serumverdünnung 1:4 Extinktion 546 nm | 295 | 99 | 215 | 165 |

Die Ergebnisse zeigen deutlich den antigenspezifischen Nachweis von Immunkomplexen, speziell bei Immunkomplexen im leichten Antigenüberschuss.

## Patentansprüche

1. Immunologisches Bestimmungsverfahren für einen Immunkomplex in einer Flüssigkeit, dadurch gekennzeichnet, dass

a) ein für eine antigene Determinante des Antigens in dem Immunkomplex spezifischer, an einen Träger gebundener Reaktant A in einer für die Bindung des Immunkomplexes ausreichenden Menge mit der immunkomplexhaltigen Flüssigkeit inkubiert wird;

b) der so behandelte Träger nach Abtrennung der Flüssigkeit mit einer für die Bindung an den Immunkomplex ausreichenden Menge einer Lösung eines gegebenenfalls markierten Reaktanten B inkubiert wird, der spezifisch für eine antigene Determinante des Antikörpers in dem Immunkomplex ist;

c) und nach Trennung von Träger und Flüssigkeit durch Messung der an den Träger gebundenen oder der in der Flüssigkeit verbleibenden Menge des Reaktanten B der Immunkomplex bestimmt wird.

2. Verfahren nach Anspruch 1,dadurch gekennzeichnet, dass der Träger ein biologisches Partikel oder ein organischer oder anorganischer Formkörper ist.

3. Verfahren nach Anspruch 1, dadurch gekennzeichnet, dass die Bindung des Reaktanten kovalent oder adsorptiv erfolgt.

4. Verfahren nach einem der Ansprüche 1 bis 3, dadurch gekennzeichnet, dass der mit dem Antikörper reagierende Reaktant B ein Markierungsmittel trägt.

5. Verfahren nach einem der Ansprüche 1 bis 4, dadurch gekennzeichnet, dass der mit dem Antikörper reagierende Reaktant ein Antikörper oder Antikörperfragment ist.

6. Verfahren nach einem der Ansprüche 1 bis 4, dadurch gekennzeichnet, dass der mit dem Antikörper reagierende Reaktant mikrobielles Protein A ist

7. Verfahren nach einem der Ansprüche 1 bis 4, dadurch gekennzeichnet, dass der mit dem Antikörper reagierende Reaktant ein Komplementfaktor ist.

8. Verfahren nach Anspruch 7, dadurch gekennzeichnet, dass der mit dem Antikörper reagierende Reaktant ein gegen Komplement gerichteter Antikörper oder ein Antikörperfragment ist.

9. Verfahren nach einem der Ansprüche 1 bis 5, dadurch gekennzeichnet, dass der mit dem Antikörper reagierende Reaktant ein Antikörper gegen Fc-Fragment von Immunglobulin oder gegen Immunglobulinaggregate oder ein Rheumafaktor ist.

10. Verfahren nach Anspruch 4, dadurch gekennzeichnet, dass das Markierungsmittel eine radioaktive Substanz, ein Enzym, ein Coenzym oder eine fluoreszierende Substanz ist.

## Revendications

1. Procédé de détermination immunologique pour un immuncomplexe dans un liquide, caractérisé en ce que:

a) on incube un réactif A fixé sur un support, spécifique pour un déterminant antigénique de l'antigène dans l'immuncomplexe, avec le liquide contenant l'immuncomplexe, en une quantité suffisante pour la fixation de l'immuncomplexe;

b) on incube le support ainsi traité, après séparation du liquide, avec une quantité suffisante pour la fixation sur l'immuncomplexe d'une solution d'un réactif B éventuellement marqué, qui est spécifique pour un déterminant antigénique de l'anticorps dans l'immuncomplexe;

c) et après séparation du support et du liquide on détermine l'immuncomplexe par la mesure de la quantité de réactif B fixée sur le support ou de la quantité de réactif B restant dans le liquide.

2. Procédé selon la revendication 1, caractérisé en ce que le support est une particule biologique ou une matière en forme organique ou minérale.

3. Procédé selon la revendication 1, caractérisé en ce que la fixation du réactif a lieu par covalence ou adsorption.

4. Procédé selon l'une quelconque des revendications 1 à 3, caractérisé en ce que le réactif B réagissant avec l'anticorps porte un agent de marquage.

5. Procédé selon l'une quelconque des revendications 1 à 4, caractérisé en ce que le réactif réagissant avec l'anticorps est un anticorps ou un fragment d'anticorps.

6. Procédé selon l'une quelconque des revendications 1 à 4, caractérisé en ce que le réactif réagissant avec l'anticorps est une protéine microbienne A.

7. Procédé selon l'une quelconque des revendications 1 à 4, caractérisé en ce que le réactif réagissant avec l'anticorps est un facteur du complément.

8. Procédé selon la revendication 7, caractérisé en ce que le réactif réagissant avec l'anticorps est un anticorps ou un fragment d'anticorps opposé au complément.

9. Procédé selon l'une quelconque des revendications 1 à 5, caractérisé en ce que le réactif réagissant avec l'anticorps est un anticorps opposé au fragment Fc de l'immunoglobuline ou opposé aux agrégats d'immunoglobulines ou un facteur rhumatoîde.

10. Procédé selon la revendication 4, caractérisé en ce que l'agent de marquage est une substance radioactive, une enzyme, une coenzyme ou une substance fluorescente.

**Claims**

1. Process for the immunological determination of an immunocomplex in a liquid, which comprises

a) incubating a reagent A bound onto a carrier and specific for an antigenic determinant of the antigen, in a quantity which is sufficient for fixing the immunocomplex, with the immuno-complex-containing liquid,

b) incubating the carrier so treated, after separation of the liquid, with a quantity sufficient for the fixation onto the immuno-complex, of a solution of a reagent B, which may be labelled and which is specific for an antigenic determinant of the antibody in the immuno-complex and

c) after separating the carrier from the liquid determining the immuno-complex by measuring the reagent B bound onto the carrier or remaining in the liquid.

2. A process as claimed in claim 1, wherein the carrier is a biological particle or an organic or inorganic shaped body.

3. A process as claimed in claim 1, wherein the fixation of the reagent is effected covalently or adsorptively.

4. A process as claimed in any one of claims 1 to 3, wherein the reagent B which reacts with the antibody carries a labelling agent.

5. A process as claimed in any one of claims 1 to 4, wherein the reagent which reacts with the antibody is an antibody or antibody fragment.

6. A process as claimed in any one of claims 1 to 4, wherein the reagent which reacts with the antibody is microbial protein A.

7. A process as claimed in any one of claims 1 to 4, wherein the reagent which reacts with the antibody is a complement factor.

8. A process as claimed in claim 7, wherein the reagent which reacts with the antibody is an antibody or antibody fragment directed against the complement.

9. A process as claimed in any one of claims 1 to 5, wherein the reagent which reacts with the antibody is an antibody against Fc-fragment of immunoglobulin or against immunoglobulin aggregates or a rheuma factor.

10. A process as claimed in claim 4, wherein the labelling agent is a radio-active substance, an enzyme, a coenzyme or a fluorescent substance.